Europäisches Patentamt

European Patent Office

Office européen des brevets

(11) Numéro de publication: **0 404 661**
**A2**

## (12) DEMANDE DE BREVET EUROPEEN

(21) Numéro de dépôt: **90401723.3**

(22) Date de dépôt: **19.06.90**

(51) Int. Cl.5: **A61K 37/20**

(30) Priorité: **20.06.89 FR 8908195**

(43) Date de publication de la demande:
**27.12.90 Bulletin 90/52**

(84) Etats contractants désignés:
**CH DE GB IT LI NL**

(71) Demandeur: **ROUSSEL-UCLAF**
**35, boulevard des Invalides**
**F-75007 Paris(FR)**

(72) Inventeur: **Bonfils, Armelle**
**21, rue A. Crapotte**
**F-78700 Conflans Sainte Honorine(FR)**
Inventeur: **Smets, Pierre**
**137, Avenue du Maréchal Leclerc**
**F-78670 Villennes sur Seine(FR)**
Inventeur: **Zalisz. René**
**20, rue Delattre de Tassigny**
**F-95180 Menucourt(FR)**

(74) Mandataire: **Fritel, Hubert et al**
**111, route de Noisy B.P. no 9**
**F-93230 Romainville(FR)**

(54) **Utilisation de lipopolysaccharides extraits de bactéries gram (-) pour la fabrication d'un médicament facilitant la cicatrisation de la peau.**

(57) Utilisation de lipopolysaccharides extraits de bactéries gram (-), pour la fabrication d'un médicament facilitant la cicatrisation de la peau.

EP 0 404 661 A2

# Utilisation de lipopolysaccharides extraits de bactéries gram (-) pour la fabrication d'un médicament facilitant la cicatrisation de la peau.

La présente demande a pour objet l'utilisation de lipopolysaccharides extraits de bactéries gram (-) pour la fabrication d'un médicament facilitant la cicatrisation de la peau.

Les lipopolysaccharides extraits de bactéries gram (-) ont été décrits dans la littérature en raison de leurs propriétés immuno-modulatrices ; ils présentent notamment des propriétés stimulantes non spécifiques des défenses de l'organisme (1985, Int. Archs Allergy Appl. Immun. 76 Suppl. 1. 119-127 et Journal of Immunopharmacology 3(2), 119-132 (1981).

En poursuivant ses études sur les produits précités, la demanderesse vient de trouver de façon tout à fait inattendue que les lipopolysaccharides extraits de bactéries gram (-) présentaient de très remarquables propriétés dans le domaine de la cicatrisation cutanée.

L'invention a ainsi pour objet l'utilisation de lipopolysaccharides extraits de bactéries gram (-), pour la fabrication d'un médicament facilitant la cicatrisation de la peau.

Parmi les lipopolysaccharides extraits de bactéries gram -on retient notamment les lipopolysaccharides extraits des bactéries gram (-) choisies dans le groupe formé par Klebsiella pneumoniae, Hafnia, Entérobacter cloacae, Escherichia Coli, Klebsiella Ozoenae, Pseudomonas aeruginosa.

Selon l'invention, les lipopolysaccharides tels que définis ci-dessus, présentent de remarquables propriétés cicatrisantes dans le traitement des brûlures, en chirurgie plastique et en alopécie. L'invention a ainsi pour objet l'utilisation des lipopolysaccharides tels que définis ci-dessus pour la fabrication d'un médicament facilitant la cicatrisation cutanée dans le traitement des brûlures de toutes origines, dans le traitement des ulcères de la peau, en chirurgie et en alopécie.

Parmi les lipopolysaccharides extraits de Klebsiella pneumoniae on retient de préférence les lipopolysaccharides extraits de la souche de Klebsiella pneumoniae déposée à l'Institut Pasteur à Paris sous le n° I-163.

Parmi les lipopolysaccharides extraits d'Hafnia, on retient de préférence les lipopolysaccharides extraits de la souche d'Hafnia déposée à l'Institut Pasteur à Paris sous le n° I.868.

Parmi les lipopolysaccharides extraits d'Entérobacter cloacae, on retient de préférence les lipopolysaccharides extraits de la souche d'Entérobacter cloacae déposée à l'Institut Pasteur à Paris sous le n° I.869.

Parmi les lipopolysaccharides extraits d'Escherichia coli, on retient de préférence les lipopolysaccharides extraits de la souche d'Escherichia Coli déposée à l'Institut Pasteur à Paris sous le n° I.870.

Parmi les lipopolysaccharides extraits de Klebsiella Ozoenae, on retient de préférence les lipopolysaccharides extraits de la souche de Klebsiella Ozoenae déposée à l'Institut Pasteur à Paris sous le n° I.871.

Parmi les lipopolysaccharides extraits de Pseudomonas aeruginosa, on retient de préférence les lipopolysaccharides extraits des souches de Pseudomonas aeruginosa déposées à l'ATCC sous le n° 21630 et à l'Institut Pasteur à Paris sous les n° I.872, I.873, I.874.

En étudiant l'activité des lipopolysaccharides tels que définis ci-dessus, on a constaté que ceux-ci présentaient une remarquable activité inductrice "like" de l'EGF (Epidermal Growth Factor) facteur de croissance épidermique sans présenter la toxicité de ce dernier. De ce fait, les lipopolysaccharides peuvent être utilisés pour faciliter la cicatrisation de la peau et de l'épiderme.

En raison de leurs remarquables propriétés cicatrisantes illustrées plus loin dans la partie expérimentale, les lipopolysaccharides selon l'invention, peuvent être utilisés dans le traitement des brûlures de toutes origines, dans le traitement des grands brûlés, des érythèmes solaires, des plaies superficielles, des gerçures, des crevasses, des engelures, des piqûres d'insectes, en chirurgie ou en chirurgie plastique, pour faciliter la cicatrisation, dans le vieillissement de la peau, enfin en alopécie.

La dose usuelle, variable selon le produit utilisé, le sujet traité et l'affection en cause, peut être, par exemple de 50 μg à 5 mg par jour en applications locales chez l'homme.

A titre de médicaments, les lipopolysaccharides de la présente demande peuvent être administrés en applications locales.

Les compositions pharmaceutiques correspondantes peuvent être, par exemple, solides ou liquides et se présenter sous les formes pharmaceutiques couramment utilisées en médecine humaine, comme par exemple, les crèmes, les gels, les pommades, les lotions, les laits ou huiles pour la peau, les gouttes, les collyres, les aérosols, les shampoings ou sous forme de liposomes ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

De nombreux procédés de préparation de lipopolysaccharides ont été décrits dans la littérature. On peut notamment se reporter à l'article de 0. Westphal et K. Jann in Methods in carbohydrate Chemistry (Whister RL Ed Vol. 5/p. 83, Academic Press, New-York, 1965).

Les procédés décrits utilisent le plus souvent une extraction par un mélange phénol/eau du culot de centrifugation de la culture microbienne suivie d'une dialyse.

Des exemples d'une telle préparation figurent plus loin dans la partie expérimentale.

Il va être donné maintenant à titre non limitatif des exemples de mise en oeuvre de l'invention.

## EXEMPLE 1 : Lipopolysaccharides Hafnia

Après centrifugation de la culture d'Enterobacter Hafnia (souche déposée à l'Institut Pasteur à Paris sous le n° I.868), le culot est lavé 2 fois par une solution de chlorure de sodium à 9 ‰ et 2 fois par de l'acétone.

Après centrifugation, le culot est mis à sécher à 40°C sous vide, remis en solution dans de l'eau distillée à 65°C et extrait avec un volume égal d'un mélange phénol/eau (90/10) à 65°C. Après refroidissement dans un bain de glace et centrifugation (10.000 t/mn, 30 minutes à 10°C), la phase aqueuse est récupérée, celle-ci est dialysée sous flux continu avec de l'eau permutée pendant 2 jours, puis lyophilisée.

On obtient ainsi 2,32 g de lipopolysaccharides d'Hafnia par litre de fermenteur.

## EXEMPLE 2 : Lipopolysaccharides de Klebsiella pneumoniae

En opérant comme à l'exemple 1, mais au départ de la souche de Klebsiella pneumoniae non capsulée déposée à l'Institut Pasteur à Paris sous le n° I-163, on a obtenu 1,33 g de lipopolysaccharides de Klebsiella pneumoniae par litre de fermenteur.

## EXEMPLE 3 : Lipopolysaccharides de Klebsiella Ozoenae

En opérant comme à l'exemple 1, mais au départ de la souche de Klebsiella Ozoenae capsulée type 4 déposée à l'Institut Pasteur à Paris sous le n° I.871, on a obtenu 1,42 g de lipopolysaccharides de Klebsiella Ozoenae par litre de fermenteur.

## EXEMPLE 4 : Lipopolysaccharides de Pseudomonas Aeruginosa

En opérant comme indiqué à l'exemple 1, mais au départ de la souche de Pseudomonas Aeruginosa déposée à l'ATCC sous le n° 21630, on a obtenu 0,6 g de lipopolysaccharides de Pseudomonas Aeruginosa par litre de fermenteur.

En opérant de la même manière, mais au départ des souches de Pseudomonas Aeruginosa déposées à l'Institut Pasteur sous les n° I.872, I.873, I.874, on a obtenu respectivement 0,8, 0,67 et 0,47 g de polysaccharides de Pseudomonas Aeruginosa par litre de fermenteurs.

## EXEMPLE 5 : pommade cicatrisante

On a préparé une pommade cicatrisante répondant à la formule :
- Lipopolysaccharides extraits de Klebsiella pneumoniae, exemple 1 g
- excipient q.s.p. 100 g

## EXEMPLE 6 : pommade cicatrisante

On a préparé une pommade cicatrisante répondant à la formule :

- Lipopolysaccharides extraits de Klebsiella pneumoniae, exemple 1 g
- excipient q.s.p. 100 g

## ETUDE DE L'ACTIVITE CICATRISANTE

### Culture

Des fibroblastes de derme humain sont multipliés en culture monocouche dans du Milieu de Eagle avec sels de Earle (EMEM, Laboratoires Boeringher) contenant 10 % de sérum de veau foetal (Laboratoires Flow). Les expériences sont effectuées avec des cultures variant de 7 à 10 passages.

Des gels tridimentionnels de 30 ml sont réalisés, en mélangeant 13,8 ml de EMEM (1.76 fois concentré), 2,7 ml de sérum de veau foetal ou de EMEM (selon les expériences), 9 ml de collagène acido-soluble extrait à partir de tendons de queues de rats (3 mg/ml dans de l'acide acétique 1/1000), 1,5 ml de NaOH 0,1N et 3 ml de suspension de fibroblastes dans du EMEM ($4 \times 10^5$ cellules/ml). Ce mélange est placé à $37°C$ dans des boîtes de Pétri de bactériologie de 100 mm de diamètre pour permettre la polymérisation du collagène et la formation d'un gel dans lequel les fibroblastes sont répartis en trois dimensions.

Des biopsies circulaires de 1 mm de diamètre sont prélevées dans des fragments de peau saine provenant de chirurgie réparatrice (plasties mammaires de femmes âgées de 20 à 45 ans). Selon les expériences ces biopsies sont implantées au centre des dermes équivalents dès la formation du gel, ou collées par une goutte de collagène sur des dermes dont la contraction est stabilisée (au 5ème jour). Dans le dernier cas, le collage de l'implant est réalisé soit sur dermes vivants, soit sur dermes dont les fibroblastes sont tués par choc osmotique avant l'épidermisation.

### Traitement par les lipopolysaccharides

Le lipopolysaccharide étudié est dissout au moment du traitement dans du sérum de veau foetal, de façon qu'il soit à une concentration finale dans la culture de 0,0005 ‰, 0,005 ‰, 0,05 ‰, 0,5 ‰ et 5 ‰.

Le lipopolysaccharide est ajouté à partir du 5ème jour, une fois que les fibroblastes ont remanié la matrice de collagène, et donc que le traitement par le lipopolysaccharide n'a plus d'effet sur cette fonction.

La durée totale du traitement est de 13 jours. Le milieu est renouvelé, avec ou sans lipopolysaccharides deux fois par semaine, en prenant garde à ce que les cultures soient toujours en immersion. Dans toutes les expériences les différents échantillons sont en 3 exemplaires.

### Evaluation quantitative de l'épidermisation

A la fin de chaque expérience, les peaux équivalentes sont transférées dans des boîtes de Pétri de 60 mm de diamètre, incubées 1 heure à $37°C$ dans 3 ml de milieu de culture contenant 2 uCi/ml de thymidine tritiée ($6^{-3}$ H Thymidine, 25 Ci/mM, CEA). Puis les spécimens sont incubés 30 min. à $37°C$ dans du sulfate de Bleu du Nil (Sigma) à la concentration finale de 1/10000, et rincés 15 min. à la température du laboratoire dans du NaCl 9 ‰, ce qui permet de bien visualiser l'épiderme qui s'est développé à la surface du derme équivalent à partir de l'implant initial.

La surface épidermisée est alors mesurée par planimétrie à l'aide d'un analyseur d'images (Nachet NS 1000).

L'étude est effectuée comparativement avec des cultures en absence et en présence d'EGF (20 mg/ml).

Les résultats figurant dans le tableau I ci-après sont la moyenne de 3 déterminations. Ces résultats montrent que les lipopolysaccharides tels que définis ci-dessus sont de puissants facteurs de croissance, souvent plus efficaces que l'EGF dans la stimulation de la croissance épidermique.

TABLEAU I

| Produit étudié | Contrôle sans EGF | Contrôle EGF | Lipopolysaccharides | | |
|---|---|---|---|---|---|
| | | 20 mg/ml | 0,5 µg/ml | 5 µg/ml | 50 µg/ml |
| - | 16,36 | 109,16 | | | |
| Ex. 2 | | | 59,30 | 89,05 | 135,11 |
| Ex. 1 | | | 50,07 | 88,27 | 176,19 |
| - | 24,51 | 111,44 | | | |
| Ex. 2 | | | | 73,07 | |
| Ex. 1 | | | - | 167,00 | |
| - | 13,72 | 118,81 | | | |
| Ex. 2 | | | | | 101,21 |
| Ex. 1 | | | - | | 100,95 |

**Revendications**

1) Utilisation de lipopolysaccharides extraits de bactéries gram (-), pour la fabrication d'un médicament facilitant la cicatrisation de la peau.

2) Utilisation de lipopolysaccharides selon la revendication 1, caractérisée en ce que les lipopolysaccharides sont extraits de bactéries gram (-) choisies dans le groupe formé par Klebsiella pneumoniae, Hafnia, Entérobacter cloacae, Escherichia Coli, Klebsiella Ozoenae, Pseudomonas aeruginosa.

3) Utilisation des lipopolysaccharides selon la revendication 1 ou 2, pour la fabrication d'un médicament facilitant la cicatrisation cutanée dans le traitement des brûlures de toutes origines, dans le traitement des ulcéres de la peau, en chirurgie et en alopécie.

4) Utilisation de lipopolysaccharides selon l'une quelconque des revendications 1 à 3, caractérisée en ce que les lipopolysaccharides sont extraits de la souche de Klebsiella pneumoniae déposée à l'Institut Pasteur à Paris sous le n° I-163.

5) Utilisation de lipopolysaccharides selon l'une quelconque des revendications 1 à 3, caractérisée en ce que les lipopolysaccharides sont extraits de la souche d'Hafnia déposée à l'Institut Pasteur à Paris sous le n° I.868.

6) Utilisation des lipopolysaccharides selon l'une quelconque des revendications 1 à 3, caractérisée en ce que les lipopolysaccharides sont extraits de la souche d'Entérobacter cloacae déposée à l'Institut Pasteur à Paris sous le n° I.869.

7) Utilisation des lipopolysaccharides selon l'une quelconque des revendications 1 à 3, caractérisée en ce que les lipopolysaccharides sont extraits de la souche d'Escherichia Coli déposée à l'Institut Pasteur à Paris sous le n° I.870.

8) Utilisation des lipopolysaccharides selon l'une quelconque des revendications 1 à 3, caractérisée en ce que les lipopolysaccharides sont extraits de la souche de Klebsiella Ozoenae déposée à l'Institut Pasteur à Paris sous le n° I.871.

9) Utilisation des lipopolysaccharides selon l'une quelconque des revendications 1 à 3, caractérisée en ce que les lipopolysaccharides sont extraits des souches de Pseudomonas aeruginosa déposées à l'ATCC sous le n° 21630 et à l'Institut Pasteur à Paris sous les n° I.872, I.873, I.874.